# EUROPEAN PATENT SPECIFICATION

(11) **EP 2 156 849 B1**
(45) Date of publication and mention of the grant of the patent: **25.10.2017**
(21) Application number: 08715072.8
(22) Date of filing: 31.03.2008
(51) Int. Cl.: A61L 15/28, D01F 9/00, D06M 11/38, D06M 13/08, D06M 13/144, D06M 13/165, D06M 13/188, D01D 5/06

(54) **A CHITOSAN BASED FIBER MATERIAL, PREPARING METHOD AND APPLICATION THEREOF**
FASERMATERIAL AUF CHITOSANBASIS, HERSTELLUNGSVERFAHREN UND SEINE ANWENDUNG
MATERIAU FIBREUX A BASE DE CHITOSANE, PROCEDE DE PREPARATION ET APPLICATION ASSOCIES

(30) Priority: 13.05.2007 CN 200710015743
(43) Date of publication of application: 24.02.2010
(73) Proprietor: Qingdao Biotemed Biomaterial Co., Ltd, Qingdao Shandong 266061 (CN)
(72) Inventor: LIU, Wanshun, Qingdao, Shandong 266003 (CN); HAN, Baoqin, Qingdao, Shandong 266003 (CN); GU, Qisheng, Shanghai 200061 (CN)
(74) Representative: Michalski Hüttermann & Partner Patentanwälte mbB
(86) International application number: PCT/CN2008/000636
(87) International publication number: WO 2008/138202

(56) References cited:
- WO-A1-2006/097024
- CN-A- 1 715 465
- CN-A- 1 920 129
- CN-A- 101 049 513
- CN-A- 101 053 669
- US-A- 5 460 939
- US-A1- 2004 166 307
- DATABASE WPI Week 200644 Thomson Scientific, London, GB; AN 2006-424707 XP002589889 -& CN 1 715 465 A 4 January 2006 (2006-01-04)
- EAST G C ET AL: "WET SPINNING OF CHITOSAN AND THE ACETYLATION OF CHITOSAN FIBERS" JOURNAL OF APPLIED POLYMER SCIENCE, JOHN WILEY AND SONS INC. NEW YORK, US LNKD- DOI:10.1002/APP.1993.070501013, vol. 50, no. 10, 10 December 1993 (1993-12-10), pages 1773-1779, XP000464421 ISSN: 0021-8995
- SUN L ET AL: "Preparation, characterization and antimicrobial activity of quaternized carboxymethyl chitosan and application as pulp-cap" POLYMER, ELSEVIER SCIENCE PUBLISHERS B.V, GB LNKD- DOI:10.1016/J.POLYMER.2006.01.073, vol. 47, no. 6, 8 March 2006 (2006-03-08), pages 1796-1804, XP025231492 ISSN: 0032-3861 [retrieved on 2006-03-08]
- SHI Y. ET AL.: 'Study on the Properties of Water-Soluble Hydroxypropyl Chitosan' JOURNAL OF SICHUAN UNIVERSITY (ENGINEERING SCIENCE EDITION) vol. 38, no. 3, May 2006, XP008123797
- FAN L ET AL: "Preparation and properties of alginate/carboxymethyl chitosan blend fibers", CARBOHYDRATE POLYMERS, APPLIED SCIENCE PUBLISHERS, LTD. BARKING, GB, vol. 65, no. 4, 13 September 2006 (2006-09-13), pages 447-452, XP027941585, ISSN: 0144-8617 [retrieved on 2006-09-13]

## Description

### Field of the Invention

The invention relates to a medical material, more particularly to a hemostatic and wound-healing water-soluble chitosan-based fiber material, its preparation method and use.

### Background of the Invention

Hemorrhage control, hemostasis and promotion of incision healing during and after clinical operation are necessary and important for shortening operation duration and reducing occurrence of complication after operation. Conventional hemostasis methods, such as ligation, suture and electrocoagulation, are commonly used for clinical hemostasis and trauma hemostasis, but are not always effective for hemorrhage control, especially in the operation of parenchymal viscera containing many blood vessels. Therefore, many kinds of topical hemostatic materials have been put into clinical application according to particular clinical requirements, including glutin sponge, collagen sponge, fibrin glue and oxycellulose. Glutin sponge which has been applied in clinic for many years has a weaker hydrophilicity and a relatively poor hemostatic effect for massive hemorrhage. Collagen sponge which has a better hemostatic effect than glutin sponge is obtained from bovin achilles tendon and tendon, and therefore is exposed to a risk of spreading animal viral diseases. Fibrin glue has a wide application in the clinical operation hemostasis. Because fibrin glue components belong to blood products, however, fibrin glue is exposed to a risk of spreading antigenic diseases and viral diseases, and therefore is doubted for its application safety. The glutin sponge, collagen sponge and fibrin glue as mentioned above all belong to heterogeneous animal proteins, have certain immunogenicity and are exposed to a risk of spreading virus, while increasing chances of wound infection. Hemostatic materials made from oxycellulose have been applied to topical hemostasis in various clinical operations, such as neurosurgery, abdominal surgery, and thoracic surgery, with preference given to products sold by Johnson & Johnson, including Surgicel ○,R absorbable hemostatic, Surgicel Nu-Knit ○,R absorbable hemostatic, and Surgicel ○,R Fibrllar absorbable hemostatic. However, the oxycellulose hemostatic material has a good hemostatic effect for slight blood exudation; in contrast, it exhibits slow hemostasis for serious blood exudation, and the effect is not quite ideal. Up to now a hemostatic material having a satisfactory hemostasis effect for serious blood exudation has not been developed. The hemostatic materials as mentioned above do not provide a promotive effect on incision healing or a satisfactory promotive effect on traumatic healing, while exerting a hemostatic effect.

Chitosan, a linear polysaccharide, is a positively charged, non-immunogenical and biodegradable product from chitin by removal of acetyl, which has many physiological activities, for example, promotion of wound healing, hemostasis and analgesic effect, antibacterial and bacteriostatic effect. The hemostatic effect of chitosan mainly results from the interaction between the charges of chitosan and erythrocyte and coagulation. However, the application of chitosan is greatly limited, especially in medical material, due to its dissolubility in water. Chitosan powder, chitosan membrane, chitosan sponge, and chitosan non-woven and woven fabrics obtained from chitosan degrade slowly in human body, and the complete degradation thereof takes over half a year. Many research shows that chitosan causes a systematic inflammatory reaction during degradation in the body. Therefore, hemostatic material and wound-healing material made from chitosan are only applicable to skin wound.

A carboxymethyl chitosan fiber and its preparation method and use are described in Chinese Patent No. ZL200410025721.4. The polymeric monomer of the carboxymethyl chitosan fiber has a degree of substitution of carboxyl of 1 to 50%, imparting the fiber a high hygroscopicity while maintaining a fibrous structure and thus its dissolubility in water. However, the carboxymethyl chitosan fiber with a low In the article of East and Qin from 1993, a process for the manufacture of chitosan fibers by wet spinning is described. Specifically, the fibers were produced by a wet spinning process and were subsequently acetylated to generate chitin fibers. degree of substitution takes a long time for degradation in body, and also would cause a systematic inflammatory reaction, and therefore is not applicable to body.

### Disclosure of the Invention

The object of the invention is to provide a water-soluble chitosan-based fiber material and its preparation method and use to overcome the above drawbacks.

According to one aspect of the invention, there is provided a water-soluble chitosan-based fiber material, as specified in claim 1, wherein the water-soluble chitosan-based fiber comprises carboxymethyl chitosan fiber, hydroxypropyl chitosan fiber, hydroxyethyl chitosan fiber, carboxymethyl hydroxyethyl chitosan fiber or mixtures thereof. The water-soluble chitosan-based fiber material according to the invention may be in the form of fiber, sliver, sponge, cotton wool, non-woven fabric, knitted fabric, braided fabric, woven fabric, etc.

According to another aspect of the invention, there is provided a method of preparing the water-soluble chitosan-based fiber material, as specified in claim 3, comprising the following steps:
a water-insoluble chitosan fiber material is immersed in an aqueous solution of NaOH or KOH having a concentration of 20-50% by weight, preferably 30-45% by weight, a weight ratio of the water-insoluble chitosan fiber material to the aqueous solution being 1: (5-40), preferably 1: (10-25), and alkalized at a temperature ranging from -40°C to room temperature for 2 to 72 hours, preferably 12 to 48 hours;
the alkalized water-insoluble chitosan-based fiber material is reacted with halogenated acetic acid, or halogenated ethanol, or propylene oxide, or halogenated propanol, or combination of halogenated acetic acid and halogenated ethanol in a reaction medium for a period of 5 to 36 hours, preferably 6 to 24 hours, to give a corresponding water-soluble chitosan fiber material;
wherein, the mole ratio of the water-insoluble chitosan-based fiber material to the alkaline to the halogenated acetic acid, or halogenated ethanol, or propylene oxide, or halogenated propanol, or combination of halogenated acetic acid and halogenated ethanol is 1: (10-40): (1.1-15), preferably 1: (20-40): (5-12).

According to another aspect of the invention, there is provided a method of preparing the water-soluble chitosan-based fiber material, comprising the following steps:
a water-soluble chitosan-based powder is dissolved in distilled water with stirring to form a viscous gel solution, and a minor amount of insoluble substance is removed by press filtering, giving a water-soluble chitosan-based spinning solution having a concentration of 2-10% by weight, preferably 5-8% by weight; the spinning solution is blown into a coagulation bath at a temperature of 30-45°C via a spinning die, to form a water-soluble chitosan-based fiber; the fiber is drawn off by a capstan roll into a stretch bath, stretched by a draw roll and taken up, to produce a water-soluble chitosan-based fiber bundle; a dried water-soluble chitosan-based fiber is obtained by removal of organic solvent and drying; after finishing, cutting, packing and sterilizing, a sliver, sponge or cotton wool of the water-soluble chitosan-based fiber material is produced; or the water-soluble chitosan-based fiber material is further processed into the form of non-woven fabric, knitted fabric, braided fabric, woven fabric.

According to still another aspect, the invention further relates to the use of the water-soluble chitosan-based fiber material in producing drugs or medical devices for hemostasis, wound healing or prevention of tissue adhesion.

The drugs or medical devices produced from the water-soluble chitosan-based fiber material are used in hemostasis, promotion of wound healing and prevention of tissue adhesion before, during and after clinical operation.

The water-soluble chitosan-based fiber material according to the invention has all the effects of hemostasis, promotion of wound-healing and prevention of tissue adhesion; is capable of swelling gradually by absorbing water and dissolving gradually to form a gel solution in the physiological pH condition, being effective for topical sticking and exerting much better effects of hemostasis, wound-healing and prevention of tissue adhesion; and is free of immunogenicity in body, degrades in a short time, has a good biocompatibility, is safe in clinical application and suitable for hemostasis in body, wound healing and prevention of tissue adhesion. The water-soluble chitosan-based fiber material according to the invention in the form of sliver, sponge, cotton wool, non-woven fabric, knitted fabric, braided fabric, woven fabric has a certain mechanical strength, is convenient to handle in clinical operation, adheres strongly, and could exert its effects better and be more suitable for clinical application.

### Preferred Embodiments of the Invention

In the water-soluble chitosan-based fiber material according to the invention, the water-soluble chitosan-based fiber is selected from carboxymethyl chitosan fiber, hydroxypropyl chitosan fiber, hydroxyethyl chitosan fiber, carboxymethyl hydroxyethyl chitosan fiber or mixtures thereof, as specified in claim 1.

More specifically, in the water-soluble chitosan-based fiber material according to the invention, the carboxymethyl chitosan may have the following structure: 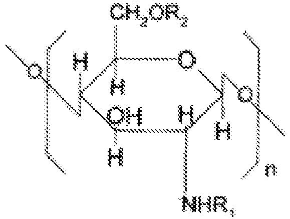 wherein, R₁ represents H, -COCH₃, -CH₂COONa or -CH₂COOK; R₂ represents H, -CH₂COONa or -CH₂COOK, and the degree of substitution of carboxymethyl is more than 60%. The carboxymethyl chitosan fiber is preferably sodium carboxymethyl chitosan fiber or potassium carboxymethyl chitosan fiber.

In the water-soluble chitosan-based fiber material according to the invention, the hydroxypropyl chitosan may have the following structure: 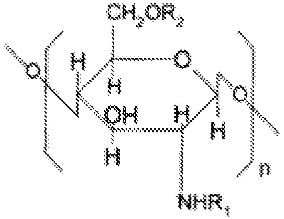 wherein, R₁ represents H, -COCH₃ or -CH₂CHOHCH₃; R₂ represents H or -CH₂CHOHCH₃, and the degree of substitution of hydroxypropyl is more than 60%.

In the water-soluble chitosan-based fiber material according to the invention, the hydroxyethyl chitosan may have the following structure: 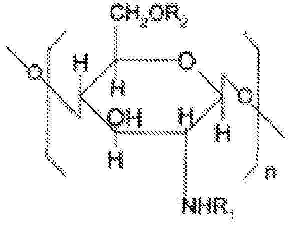 wherein, R₁ represents H, -COCH₃ or -CH₂CH₂OH; R₂ represents H or -CH₂CH₂OH, and the degree of substitution of hydroxyethyl is more than 60%.

In the water-soluble chitosan-based fiber material according to the invention, the carboxymethyl hydroxyethyl chitosan may have the following structure: 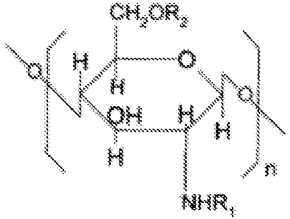 wherein, R₁ represents H, -COCH₃, -CH₂COONa, -CH₂COOK or -CH₂CH₂OH; R₂ represents H, -CH₂COONa, -CH₂COOK or -CH₂CH₂OH, and the total degree of substitution of carboxymethyl and hydroxyethyl is more than 60%.

The water-soluble chitosan-based fiber material according to the invention may be prepared by a conventional method, starting from a water-soluble chitosan-based powder, such as water-soluble carboxymethyl chitosan, hydroxypropyl chitosan, hydroxyethyl chitosan, carboxymethyl hydroxyethyl chitosan or mixtures thereof. For example, the powder of water-soluble carboxymethyl chitosan, hydroxypropyl chitosan, hydroxyethyl chitosan, carboxymethyl hydroxyethyl chitosan or mixtures thereof is dissolved in distilled water with stirring at room temperature and left to stand, to form a viscous gel solution; the solution is press filtered in a filter by using a superposition of 200 mesh, 300 mesh, 400 mesh, 500 mesh silk to remove a minor amount of insoluble substances; the filtrate is degassed under vacuum to give a clear gel solution, that is water-soluble chitosan-based spinning solution. The spinning solution is blown into a coagulation bath via a spinning die, to form a water-soluble chitosan-based fiber. The fiber is drawn off by a capstan roll into a stretch bath, stretched by a draw roll and then taken up. The resulting water-soluble chitosan-based fiber bundle is subjected to centrifugal drying to remove organic solvents, and then dried at a temperature of 50-70°C, thereby obtaining a dried water-soluble chitosan-based fiber; after finishing, cutting, packing and sterilizing, a fiber sliver, sponge or cotton wool of the water-soluble chitosan-based fiber material is produced.

The water-soluble chitosan may be in a concentration of 2-10% by weight, preferably 5-8% by weight in the spinning solution. The coagulation bath and stretch bath may be one or two of ethanol and acetone, or an aqueous solution thereof in a concentration of more than 90% by weight. The temperature of the coagulation bath may be between 30°C and 45°C. ⁶⁰Co sterilization or ethylene oxide sterilization is used for the sterilization.

The water-soluble chitosan-based fiber material obtained as above may be further processed into the form of non-woven fabric, knitted fabric, braided fabric, woven fabric by a method known to a person skilled in the art.

In the invention, non-woven fabric is a sheet, fiber web or wadding made from oriented or randomly oriented fiber bonded together by friction, cohesion or adhesion, or combination thereof. Woven fabric is produced by interlacing regularly two sets of yarns aligned respectively in the traverse and machine direction in a loom. Knitted fabric is produced by intertwining yarns or filaments in a series of loops pulled through each other by knitting needle. Braided fabric is produced by interweaving yarns or threads.

In another aspect, the water-soluble chitosan-based fiber material according to the invention may be prepared from a water-insoluble chitosan-based fiber by a method comprising the following steps: a water-insoluble chitosan-based fiber is immersed in an aqueous solution of NaOH or KOH having a concentration of 20-50% by weight in a weight ratio of 1: (5-40), and alkalized at a temperature ranging from -40°C to room temperature for 2 to 72 hours; the alkalized water-insoluble chitosan-based fiber is transferred into a reaction medium and a reaction reagent, halogenated acetic acid, or halogenated ethanol, or propylene oxide, or halogenated propanol, or combination of halogenated acetic acid and halogenated ethanol, is added with agitating at room temperature, the mole ratio of the water-insoluble chitosan-based fiber material to the base to the halogenated acetic acid, or halogenated ethanol, or propylene oxide, or halogenated propanol, or combination of halogenated acetic acid and halogenated ethanol being 1: (10-40): (1.1-15); after reaction for a period of 5 to 36 hours, the resulting solid is washed by a washing solution until neutral, dehydrated and freed of excess liquids, and then dried in a vacuum drier containing P₂O₅, subjected to evaporation to remove residual organic solvent; thereafter finishing, cutting, packing and sterilizing are carried out.

A water-insoluble chitosan fiber non-woven fabric, chitosan fiber, chitosan fiber woven fabric, chitosan fiber braided fabric can all be referred to as water-insoluble chitosan-based fiber. In the method according to the invention, the water-insoluble chitosan-based fiber may be one of chitosan fiber, chitosan fiber non-woven fabric, chitosan fiber knitted fabric, chitosan fiber braided fabric and chitosan fiber woven fabric.

The reaction medium may be one, two or three of methanol, ethanol, isopropanol, butanol, acetone, dimethylsulfoxide, tetrahydrofuran and dioxane, or an aqueous solution thereof.

The reaction reagent may be one of halogenated acetic acid, or one of halogenated ethanol, or propylene oxide, or one of halogenated propanol, or one of combination of halogenated acetic acid and halogenated ethanol. The halogenated acetic acid is chloracetic acid, bromacetic acid or iodoacetic acid. The halogenated ethanol is chlorethanol or bromethanol. The halogenated propanol is 1-chloro-2-propanol or 1-bromo-2-propanol. The combination of halogenated acetic acid and halogenated ethanol is chloracetic acid and chlorethanol, bromacetic acid and chlorethanol, chloracetic acid and bromethanol, or bromacetic acid and bromethanol.

The washing solution may be one, two or three of methanol, ethanol, isopropanol, butanol and acetone, or an aqueous solution thereof having a concentration of more than 75% by weight.

⁶⁰Co sterilization or ethylene oxide sterilization may be used for sterilizing.

The aqueous solution of the alkaline may have a concentration of 20-50% by weight, preferably 30-45% by weight. The weight ratio of the water-insoluble chitosan-based fiber to the aqueous solution may be 1 : (5-40), preferably 1 : (10-25). The alkalization may be performed at a temperature of -40°C to room temperature for a period of 2-72 hours, preferably 12-48 hours. The mole ratio of the water-insoluble chitosan-based fiber, the alkaline and the reaction reagent is 1 : (10-40) : (1.1-15), preferably 1 : (20-40) : (5-12). The reaction may be performed for a period of 5-36 hours, preferably 6-14 hours, at room temperature.

In the method according to the invention, all the water-insoluble chitosan-based fiber, in the molecular structure of which the mole percentage of deacetylated glycosyl is between 0 and 100% of the total glycosyl, may achieve the same effects.

The degree of substitution of carboxymethyl for the water-soluble carboxymethyl chitosan fiber according to the invention depends on amount of the reaction reagent in mole, reaction time and temperature during the reaction, and the substitution of carboxymethyl mainly occurs on the position of R₂, while partly on the position of R₁. The reaction condition as recited above should be selected in such a way that a degree of substitution of carboxymethyl for the water-soluble carboxymethyl chitosan fiber more than 60% is achieved eventually.

The degree of substitution of hydroxypropyl for the water-soluble hydroxypropyl chitosan fiber according to the invention depends on amount of the reaction reagent in mole, reaction time and temperature during the reaction, and the substitution of hydroxypropyl mainly occurs on the position of R₂, while partly on the position of R₁. The reaction condition as recited above should be selected in such a way that a degree of substitution of hydroxypropyl for the water-soluble hydroxypropyl chitosan fiber more than 60% is achieved eventually.

The degree of substitution of hydroxyethyl for the water-soluble hydroxyethyl chitosan fiber according to the invention depends on amount of the reaction reagent in mole, reaction time and temperature during the reaction, and the substitution of hydroxyethyl mainly occurs on the position of R₂, while partly on the position of R₁. The reaction condition as recited above should be selected in such a way that a degree of substitution of hydroxyethyl for the water-soluble hydroxyethyl chitosan fiber more than 60% is achieved eventually.

The degree of substitution of carboxymethyl and hydroxyethyl for the water-soluble carboxymethyl hydroxyethyl chitosan fiber according to the invention depends on amount of the reaction reagent in mole, reaction time and temperature during the reaction, and the substitution of carboxymethyl and hydroxyethyl mainly occurs on the position of R₂, while partly on the position of R₁. The reaction condition as recited above should be selected in such a way that a degree of substitution of carboxymethyl and hydroxyethyl for the water-soluble carboxymethyl hydroxyethyl chitosan fiber more than 60% is achieved eventually.

The water-soluble chitosan-based fiber material according to the invention feels soft, looks white, and has a good water solubility.

The following examples are given to explain the invention in more details. It is to be noted that numerous other modifications can be made by a person skilled in the art according to the invention described herein without departing from the scope of the invention. In the following examples, all temperature is in Celsius without calibration. And, unless otherwise indicated, all percentages of degree of substitution are based on mole, and the mole percentage is defined to be an average mole number of carboxymethyl or hydroxypropyl or hydroxyethyl or carboxymethyl and hydroxyethyl linked by each glycosyl. And other percentages are based on weight.

In the following examples, carboxymethyl chitosan sodium is produced by Boyite Biomaterial Company, Qingdao; and chitosan fiber, chitosan fiber non-woven fabric and chitosan fiber knitted fabric are commercially available from University of Donghua, Shanghai.

### Example 1: preparation of a water-soluble carboxymethyl chitosan fiber non-woven fabric

12g of chitosan fiber non-woven fabric having an average weight of 20.5mg/cm² is weighed into a 500ml wide-necked flask, and 200g of 45% aqueous KOH solution is added with stirring, so that chitosan fiber non-woven fabric is fully immersed into the alkaline solution. The flask with the content therein is placed at a temperature of -20°C for 24 hours and the content is alkalized. The resulting alkalized chitosan fiber non-woven fabric is transferred into a 500ml wide-necked flask, and 180g of 55% aqueous acetone solution is added as a reaction medium, and 85g of 50% monochloroacetic acid solution in ethanol is added as a reaction reagent with agitating. After reaction for 9 hours at room temperature, carboxymethyl chitosan potassium fiber non-woven fabric is obtained. The carboxymethyl chitosan potassium fiber non-woven fabric is washed sufficiently with 60% aqueous acetone solution till neutral, dehydrated with anhydrous acetone, then subjected to pressing or centrifugal drying to remove excess liquid, and dried in a vacuum drier containing P₂O₅. Then residual organic solvent is removed from the dried product by evaporation at a temperature of 50°C; thereby obtaining a carboxymethyl chitosan fiber non-woven fabric having a degree of substitution of carboxymethyl of 92%. Thereafter finishing, cutting, packing and ⁶⁰Co sterilization are carried out, to give a water-soluble chitosan-based fiber material.

### Example 2: preparation of a water-soluble carboxymethyl chitosan fiber

5g of chitosan fiber having a length of 10cm is weighed into a 500ml wide-necked flask, and 160g of 30% aqueous NaOH solution is added with stirring, so that chitosan fiber is fully immersed into the alkaline solution. The flask with the content therein is placed at room temperature for 48 hours and the content is alkalized. The resulting alkalized chitosan fiber is transferred into a 500ml wide-necked flask, and 160g of methanol is added as a reaction medium, and 46g of 50% monochloroacetic acid solution in methanol is added as a reaction reagent with agitating. After reaction for 8 hours at room temperature, carboxymethyl chitosan sodium fiber is obtained. The carboxymethyl chitosan sodium fiber is washed sufficiently with methanol till neutral, then subjected to pressing or centrifugal drying to remove excess liquid, and dried in a vacuum drier containing P₂O₅. Then residual organic solvent is removed from the dried product by evaporation at a temperature of 50°C; thereby obtaining a carboxymethyl chitosan fiber having a degree of substitution of carboxymethyl of 96%. Thereafter finishing, cutting, packing and propylene oxide sterilization are carried out, to give a water-soluble chitosan-based fiber material.

The water-soluble chitosan-based fiber material prepared according to the invention may be in the form of sliver, sponge or cotton wool of carboxymethyl chitosan fiber.

### Example 3: preparation of a water-soluble carboxymethyl chitosan fiber knitted fabric

10g of chitosan fiber knitted fabric having an average weight of 15.6mg/cm² is weighed into a 500ml wide-necked flask, and 200g of 40% aqueous NaOH solution is added with stirring, so that chitosan fiber knitted fabric is fully immersed into the alkaline solution. The flask with the content therein is placed at a temperature of 0-4°C for 48 hours and the content is alkalized. The resulting alkalized chitosan fiber knitted fabric is transferred into a 500ml wide-necked flask, and 135g of 90% aqueous ethanol solution and 50g of dimethyl sulfoxide are added as reaction medium, and 70g of 50% monochloroacetic acid solution in ethanol is added as a reaction reagent with agitating. After reaction for 10 hours at room temperature, carboxymethyl chitosan sodium fiber knitted fabric is obtained. The carboxymethyl chitosan sodium fiber knitted fabric is washed sufficiently with 75% aqueous ethanol solution till neutral, dehydrated with anhydrous ethanol, then subjected to pressing or centrifugal drying to remove excess liquid, and dried in a vacuum drier containing P₂O₅. Then residual organic solvent is removed from the dried product by evaporation at a temperature of 70°C; thereby obtaining a carboxymethyl chitosan fiber knitted fabric having a degree of substitution of carboxymethyl of 90%. Thereafter finishing, cutting, packing and propylene oxide sterilization are carried out, to give a water-soluble chitosan-based fiber material.

The reaction reagent used in Examples 1 to 3 may be one of halogenated acetic acid, namely chloracetic acid, bromacetic acid and idoacetic acid. All of them may achieve the same effects.

### Example 4: preparation of a water-soluble hydroxypropyl chitosan fiber non-woven fabric

10g of chitosan fiber non-woven fabric having an average weight of 20.5mg/cm² is weighed into a 500ml wide-necked flask, and 200g of 30% aqueous NaOH solution is added with stirring, so that chitosan fiber non-woven fabric is fully immersed into the alkaline solution. The flask with the content therein is placed at a temperature of -20°C for 60 hours and the content is alkalized. The resulting alkalized chitosan fiber non-woven fabric is transferred into a 1000ml stainless steel reaction bottle with a sealing cap, and 150g of isopropanol, 25g of dimethyl sulfoxide and 25g of dioxane are added as reaction medium, and 45g of propylene oxide is added as a reaction reagent with agitating. The reaction bottle is sealed with the cap. After reaction for 24 hours with agitating at room temperature, hydroxypropyl chitosan fiber non-woven fabric is obtained. The hydroxypropyl chitosan fiber non-woven fabric is washed sufficiently with 80% aqueous acetone solution till neutral, dehydrated with anhydrous acetone, then subjected to pressing or centrifugal drying to remove excess liquid, and dried in a vacuum drier containing P₂O₅. Then residual organic solvent is removed from the dried product by evaporation at a temperature of 50°C; thereby obtaining a hydroxypropyl chitosan fiber non-woven fabric having a degree of substitution of hydroxypropyl of 86%. Thereafter finishing, cutting, packing and ⁶⁰Co sterilization are carried out, to give a water-soluble chitosan-based fiber material.

In the example, the reaction agent may be one of propylene oxide and halogenated propanol, namely propylene oxide, 1-chloro-2-propanol and 1-bromo-2-propanol. All of them may achieve the same effects.

### Example 5: preparation of a water-soluble hydroxyethyl chitosan fiber non-woven fabric

10g of chitosan fiber non-woven fabric having an average weight of 20.5mg/cm² is weighed into a 500ml wide-necked flask, and 200g of 45% aqueous KOH solution is added with stirring, so that chitosan fiber non-woven fabric is fully immersed into the alkaline solution. The flask with the content therein is placed at a temperature of -20°C for 48 hours and the content is alkalized. The resulting alkalized chitosan fiber non-woven fabric is transferred into a 1000ml stainless steel reaction bottle with a sealing cap, and 160g of acetone, 20g of dimethyl sulfoxide and 20g of tetrahydrofuran are added as reaction medium, and 55g of monochloroethanol is added as a reaction reagent with agitating. The reaction bottle is sealed with the cap. After reaction for 36 hours with agitating at room temperature, hydroxyethyl chitosan fiber non-woven fabric is obtained. The hydroxyethyl chitosan fiber non-woven fabric is washed sufficiently with 85% aqueous acetone solution till neutral, dehydrated with acetone, then subjected to pressing or centrifugal drying to remove excess liquid, and dried in a vacuum drier containing P₂O₅. Then residual organic solvent is removed from the dried product by evaporation at a temperature of 50°C; thereby obtaining a hydroxyethyl chitosan fiber non-woven fabric having a degree of substitution of hydroxyethyl of 91%. Thereafter finishing, cutting, packing and ethylene oxide sterilization are carried out, to give a water-soluble chitosan-based fiber material.

In the example, the reaction agent may be one of halogenated ethanol, namely chloroethanol and bromoethanol. Both of them may achieve the same effects.

### Example 6: preparation of a water-soluble carboxymethyl hydroxyethyl chitosan fiber non-woven fabric

10g of chitosan fiber non-woven fabric having an average weight of 20.5mg/cm² is weighed into a 500ml wide-necked flask, and 200g of 45% aqueous KOH solution is added with stirring, so that chitosan fiber non-woven fabric is fully immersed into the alkaline solution. The flask with the content therein is placed at a temperature of -20°C for 48 hours and the content is alkalized. The resulting alkalized chitosan fiber non-woven fabric is transferred into a 1000ml stainless steel reaction bottle with a sealing cap, and 160g of acetone, 20g of dimethyl sulfoxide and 20g of tetrahydrofuran are added as reaction medium, and 46g of monochloroacetic acid solution in ethanol and 30g of monochloroethanol are added as reaction reagents with agitating. The reaction bottle is sealed with the cap. After reaction for 48 hours with agitating at room temperature, carboxymethyl hydroxyethyl chitosan fiber non-woven fabric is obtained. The carboxymethyl hydroxyethyl chitosan fiber non-woven fabric is washed sufficiently with 85% aqueous acetone solution till neutral, dehydrated with acetone, then subjected to pressing or centrifugal drying to remove excess liquid, and dried in a vacuum drier containing P₂O₅. Then residual organic solvent is removed from the dried product by evaporation at a temperature of 50°C; thereby obtaining a carboxymethyl hydroxyethyl chitosan fiber non-woven fabric having a degree of substitution of carboxymethyl and hydroxyethyl of 94%. Thereafter finishing, cutting, packing and ethylene oxide sterilization are carried out, to give a water-soluble chitosan-based fiber material.

### Example 7

120g of water-soluble carboxymethyl chitosan sodium powder having a degree of substitution of carboxymethyl more than 60% is weighed into a 3000ml clean stainless steel container, and 2000ml deionized water is added. The mixture is stirred at room temperature and left to stand, allowing a complete dissolution of the carboxymethyl chitosan sodium powder to form a viscous gel solution. The solution is press filtered in a filter by using a superposition of 200 mesh, 300 mesh, 400 mesh, 500 mesh silk to remove a minor amount of insoluble substance; the filtrate is degassed under vacuum to give a clear gel solution of carboxymethyl chitosan, that is water-soluble chitosan spinning solution. The spinning solution is blown into a coagulation bath of 95% aqueous ethanol solution at a temperature of 40-45°C via a spinning die, to form carboxymethyl chitosan fiber. The fiber is drawn off by a capstan roll into a stretch bath of 95% aqueous ethanol solution, stretched by a draw roll and then taken up. The resulting carboxymethyl chitosan fiber bundle is subjected to centrifugal drying to remove organic solvents, and then dried at a temperature of 50-70°C, thereby obtaining carboxymethyl chitosan fiber. After finishing, cutting, packing and ethylene oxide sterilization, a sliver, sponge or cotton wool of the carboxymethyl chitosan fiber is produced.

In Example 7, the carboxymethyl chitosan sodium may be replaced with carboxymethyl chitosan potassium, hydroxypropyl chitosan or hydroxyethyl chitosan. All of them are referred to as water-soluble chitosan-based material, which has a degree of substitution of carboxymethyl, hydroxypropyl or hydroxyethyl more than 60%, and may achieve the same effects. The concentration of the water-soluble chitosan-based fiber material in the spinning solution may be 2-10% by weight, preferably 5-8%. The coagulation bath and stretch bath may be one or both of ethanol and acetone, or an aqueous solution thereof in a concentration more than 90% by weight. The temperature of the coagulation bath may be 30-45°C. Either ⁶⁰Co sterilization or ethylene oxide sterilization may be utilized.

### Example 8

The carboxymethyl chitosan fiber obtained in Example 7 is subjected to opening, carding, cutting off, lapping, backing, fleece forming, cutting and drying. The resulting carboxymethyl chitosan fiber non-woven fabric is subjected to finishing, cutting, packing and ethylene oxide sterilization, to produce a non-woven fabric of water-soluble chitosan-based fiber material.

In Example 8, the carboxymethyl chitosan fiber may be replaced with hydroxypropyl chitosan fiber or hydroxyethyl chitosan fiber, and all of them may achieve the same effects. The non-woven fabric of water-soluble chitosan-based fiber material refers to carboxymethyl chitosan fiber non-woven fabric, hydroxypropyl chitosan fiber non-woven fabric or hydroxyethyl chitosan fiber non-woven fabric.

### Example 9

The performances of the water-soluble chitosan-based fiber material with regard to hemostasis, wound healing and prevention of tissue adhesion after operation are tested and evaluated as follows:

A liver tissue defect model is produced by using a rat as a test animal, and the respective effect of an oxidized regenerated cellulose gauze (a product by Johnson & Johnson), a water-soluble carboxymethyl chitosan fiber non-woven fabric obtained in example 1, a water-soluble hydroxypropyl chitosan fiber non-woven fabric obtained in example 4 and a water-soluble hydroxyethyl chitosan fiber non-woven fabric obtained in example 5 with regard to hemostasis, wound-healing and prevention of tissue adhesion after operation for the liver tissue defect is evaluated and compared. Before the test, all of the materials are cut into pieces of 1cm * 1cm in a sterile environment. 60 SD male rats weighed 250g±20g are chosen and divided randomly into negative control group (no material), oxidized regenerated cellulose gauze control group (shortened as oxidized cellulose gauze group, in which oxidized regenerated cellulose gauze is used as the material), water-soluble chitosan-based material test group No. 1 (shortened as carboxymethyl chitosan fiber non-woven fabric group, in which carboxymethyl chitosan fiber non-woven fabric is used as the material), water-soluble chitosan-based material test group No. 2 (shortened as hydroxypropyl chitosan fiber non-woven fabric group, in which hydroxypropyl chitosan fiber non-woven fabric is used as the material) and water-soluble chitosan-based material test group No. 3 (shortened as hydroxyethyl chitosan fiber non-woven fabric group, in which hydroxyethyl chitosan fiber non-woven fabric is used as the material), with 12 rats in each group. All the rats are given an anaesthesia with pentobarbital sodium intraperitoneal injection in a dosage of 30 mg/kg. And the anaesthetized rats are laparotomized by making an incision with a length of about 3cm on median abdomen, through which left lobe of liver is externalized under sterile condition, and a liver tissue of 5mm in length * 5mm in width * 2mm in thickness is removed downside from median liver by using a operating forceps, thereby causing a liver tissue defect. After 5 seconds of haemorrhage from the removal of the liver tissue, a medical gauze is used to absorb blood, and the corresponding material of 1cm * 1cm is applied immediately. Three layers of the oxidized cellulose gauze (weight 61-63g), two layers of carboxymethyl chitosan fiber non-woven fabric (weight 59-61g), two layers of hydroxypropyl chitosan fiber non-woven fabric (weight 61-63g), two layers of hydroxyethyl chitosan fiber non-woven fabric (weight 60-62g) are applied respectively. Thereafter, 10g poise is placed on the materials and remains for 20 seconds, then traumatic bleeding is observed for 40 seconds. The above procedure is repeated and bleeding is recorded. After 3 minutes, the incision is sewed and disinfected by iodophor. The rats are raised in groups and the effects on traumatic healing for animal liver tissue defect and tissue adhesion are observed respectively on days 7 and 14.

The results are summarized in tables 1 and 2. It is shown that the three types of water-soluble chitosan-based fiber materials and the oxidized regenerated cellulose gauze, compared to the negative control group, have notable effects of hemostasis, promotion of traumatic healing and prevention of tissue adhesion; furthermore, the three types of water-soluble chitosan-based fiber materials exert better effects by reducing significantly hemostasis time and traumatic healing time, and preventing tissue adhesion after operation.

**Table 1**

| group | test animal number | Hemostasis time (seconds) |
|---|---|---|
| negative comparison | 12 | >180 |
| oxidized cellulose gauze | 12 | 80-120 |
| Carboxymethyl chitosan fiber non-woven fabric | 12 | ≤ 20 |
| hydroxypropyl chitosan fiber non-woven fabric | 12 | ≤ 30 |
| hydroxyethyl chitosan fiber non-woven fabric | 12 | ≤ 30 |

**Table 2: evaluation on the performances of the water-soluble chitosan-based fiber material with regard to promotion of traumatic healing and prevention of tissue adhesion after operation**

| group | test results after 7 days | | test results after 14 days | |
|---|---|---|---|---|
| | animal number | traumatic healing after 7 days | animal number | traumatic healing after 14 days |
| negative control* | 3 | around 20% traumatic healing and serious tissue adhesion | 4 | around 20% traumatic healing and serious tissue adhesion |
| oxidized cellulose gauze | 6 | the material substantially degraded, forming purple black gel, around 40%-50% traumatic healing, and presence of tissue adhesion | 6 | the material substantially degraded, forming purple black gel on the wound, around 80%-90% traumatic healing, and presence of tissue adhesion |
| Carboxymethyl chitosan fiber non-woven fabric | 6 | the material degraded, around 70%-80% traumatic healing, and no tissue adhesion | 6 | the material degraded, complete traumatic healing, and no tissue adhesion |
| hydroxypropyl chitosan fiber non-woven fabric | 6 | the material degraded, around 70%-80% traumatic healing, and no tissue adhesion | 6 | the material degraded, complete traumatic healing, and no tissue adhesion |
| hydroxyethyl chitosan fiber non-woven fabric | 6 | the material degraded, around 70%-80% traumatic healing, and no tissue adhesion | 6 | the material degraded, complete traumatic healing, and no tissue adhesion |

| | | | | |
|---|---|---|---|---|
| * 5 rats in the negative control group died within 24 hours of operation | | | | |

A drug or medical device can be made from the water-soluble chitosan-based fiber material according to the invention and applied to clinical abdomen operation, thorax operation, bone operation, gynecological operation, microsurgery operation, burn surgery, plastic operation, etc. The water-soluble chitosan-based fiber material may exert effectively its effects of hemostasis, prevention of traumatic healing and prevention of tissue adhesion after operation, when being applied on bleeding location with gentle pressure. Especially, the water-soluble chitosan-based fiber material according to the invention has a good biocompatibility, won't cause a systematic inflammatory reaction, has a short degradation in body, has good effects of hemostasis, wound healing and prevention of tissue adhesion after operation, and therefore is applicable to operation trauma.

The examples as described above show that the water-soluble chitosan-based fiber material according to the invention is useful in producing drug or medical device which is applicable for hemostasis, promotion of wound healing or prevention of tissue adhesion after operation.

## Claims

1. A water-soluble chitosan-based fiber material, wherein the water-soluble chitosan-based fiber comprises carboxymethyl chitosan fiber, hydroxypropyl chitosan fiber, hydroxyethyl chitosan fiber, carboxymethyl, hydroxyethyl chitosan fiber or a mixture thereof, wherein the carboxymethyl chitosan has the following structure: 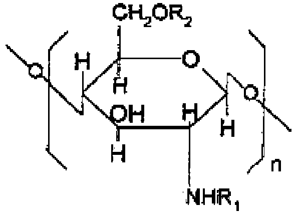 where, R₁ represents H, -COCH₃, -CH₂COONa or -CH₂COOK; R₂ represents H, -CH₂COONa or -CH₂COOK, and the degree of substitution of carboxymethyl is more than 60%;
the hydroxypropyl chitosan has the following structure: 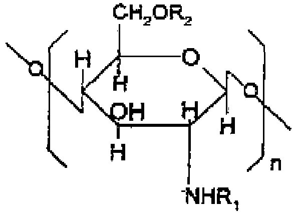 where, R₁ represents H, -COCH₃ or -CH₂CHOHCH₃; R₂ represents H or -CH₂CHOHCH₃, and the degree of substitution of hydroxypropyl is more than 60%;
the hydroxyethyl chitosan has the following structure: 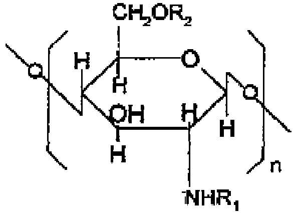 where, R₁ represents H, -COCH₃ or -CH₂CH₂OH; R₂ represents H or -CH₂CH₂OH, and the degree of substitution of hydroxyethyl is more than 60%; or
the carboxymethyl hydroxyethyl chitosan has the following structure: 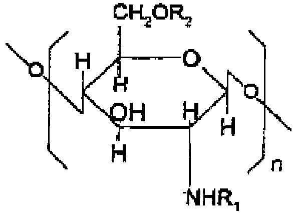 where, R₁ represents H, -COCH₃, -CH₂COONa, -CH₂COOK or -CH₂CH₂OH; R₂ represents H, -CH₂COONa, -CH₂COOK or -CH₂CH₂OH, and the total degree of substitution of carboxymethyl and hydroxyethyl is more than 60%.

2. The water-soluble chitosan-based fiber material according to claim 1 in the form of fiber, sliver, sponge, cotton wool, non-woven fabric, knitted fabric, braided fabric, woven fabric.

3. A method for preparing the water-soluble chitosan-based fiber material according to any one of claims 1-2, comprising the following steps:
a water-insoluble chitosan fiber material is immersed and alkalized in an aqueous solution of NaOH or KOH having a concentration of 20-50% by weight, preferably 30-45% by weight, with the weight ratio of the water-insoluble chitosan fiber material to the aqueous solution being 1: (5-40), preferably 1: (10-25), at a temperature of from -40°C to room temperature for 2 to 72 hours, preferably 12 to 48 hours;
the alkalized water-insoluble chitosan-based fiber material is reacted with halogenated acetic acid, or halogenated ethanol, or propylene oxide, or halogenated propanol, or combination of halogenated acetic acid and halogenated ethanol at room temperature in a reaction medium for a period of 5 to 36 hours, preferably 6 to 24 hours, to give the corresponding water-soluble chitosan fiber material,
wherein the mole ratio of the water-insoluble chitosan-based fiber material to the alkaline to the halogenated acetic acid, or halogenated ethanol, or propylene oxide, or halogenated propanol, or combination of halogenated acetic acid and halogenated ethanol is 1: (10-40): (1.1-15), preferably 1: (20-40): (5-12).

4. The method according to claim 3, wherein the halogenated acetic acid is chloracetic acid, bromacetic acid or iodoacetic acid, the halogenated ethanol is chlorethanol or bromethanol, the halogenated propanol is 1-chloro-2-propanol or 1-bromo-2-propanol, and the combination of halogenated acetic acid and halogenated ethanol is chloracetic acid and chlorethanol, bromacetic acid and chlorethanol, chloracetic acid and bromethanol, or bromacetic acid and bromethanol.

5. The method according to claim 3, wherein the reaction medium is one or more of methanol, ethanol, isopropanol, butanol, acetone, dimethyl sulfoxide, tetrahydrofuran and dioxane, or an aqueous solution thereof.

6. The method according to any one of claims 3-5, wherein the water-insoluble chitosan-based fiber is in the form of chitosan fiber, chitosan fiber non-woven fabric, chitosan fiber knitted fabric, chitosan fiber braided fabric or chitosan fiber woven fabric, in the molecular structure of which the mole percentage of deacetylated glycosyl may be between 0 and 100% of the total glycosyl.

7. A method for preparing the water-soluble chitosan-based fiber material according to any one of claims 1-2, comprising the following steps:
a water-soluble chitosan-based powder is dissolved in distilled water to give a water-soluble chitosan-based spinning solution having a concentration of 2-10% by weight, preferably 5-8% by weight;
the spinning solution is blown into a coagulation bath at a temperature of 30-45°C via a spinning die, to form a water-soluble chitosan-based fiber;
the fiber is drawn off by a capstan roll into a stretch bath, stretched by a draw roll and taken up, to produce a water-soluble chitosan-based fiber bundle;
a dried water-soluble chitosan-based fiber is obtained by removal of organic solvent and drying.

8. The method according to claim 7, wherein the water-soluble chitosan-based powder may be powder of carboxymethyl chitosan, hydroxypropyl chitosan, hydroxyethyl chitosan, carboxymethyl hydroxyethyl chitosan or a mixture thereof, and the degree of substitution of carboxymethyl, hydroxypropyl or hydroxyethyl is more than 60%.

9. The method according to claim 7, wherein the coagulation bath and the stretch bath may be one or both of ethanol and acetone, or an aqueous solution thereof having a concentration more than 90% by weight.

10. Use of the water-soluble chitosan-based fiber material according to any one of claims 1-2 in producing a drug or medical device for hemostasis, wound healing or prevention of tissue adhesion.

## Patentansprüche

1. Wasserlösliches Chitosan-basiertes Fasermaterial, wobei die wasserlösliche Chitosan-basierte Faser Carboxymethylchitosan-Faser, Hydroxypropylchitosan-Faser, Hydroxyethylchitosan-Faser, Carboxymethylhydroxyethylchitosan-Faser oder ein Gemisch davon aufweist, wobei das Carboxymethylchitosan die folgende Struktur aufweist: 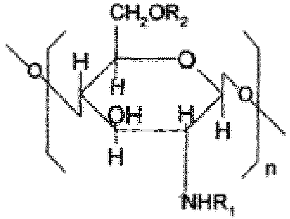 wobei R₁ für H, -COCH₃, -CH₂COONa oder -CH₂COOK steht, R₂ für H, -CH₂COONa oder -CH₂COOK steht und der Grad an Carboxymethyl-Substitution höher als 60% liegt, das Hydroxypropylchitosan folgende Struktur hat: 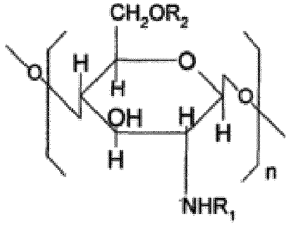 wobei R₁ für H, -COCH₃ oder -CH₂CHOHCH₃ steht, R₂ für H oder -CH₂CHOHCH₃ steht und der Grad an Carboxymethyl-Substitution höher als 60% liegt,
das Hydroxyethylchitosan folgende Struktur hat: 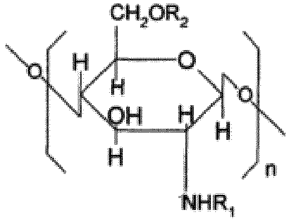 wobei R₁ für H, -COCH₃ oder -CH₂CH₂OH steht, R₂ für H oder -CH₂CH₂OH steht und der Grad an Carboxymethyl-Substitution höher als 60% liegt oder
das Carboxymethylhydroxyethylchitosan die folgende Struktur hat 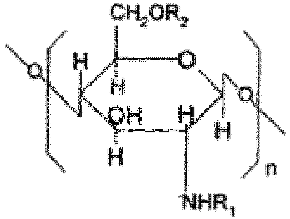 wobei R₁ für H, -COCH₃, -CH₂COONa, -CH₂COOK oder -CH₂CH₂OH steht, R₂ für H, -CH₂COONa, -CH2COOK oder -CH₂CH₂OH steht und der Gesamtgrad der Substitution von Carboxymethyl- und Hydroxyethyl-Substitution höher als 60% liegt.

2. Wasserlösliches Chitosan-basiertes Fasermaterial nach Anspruch 1 in Form von Faser, Faserband, Schwamm, Watte, Vliesstoff, Maschenware, geflochtenem Gewebe oder Gewebetuch.

3. Verfahren zum Herstellen des wasserlöslichen Chitosan-basierten Fasermaterials nach einem der Ansprüche 1 bis 2, das die folgenden Schritte aufweist:
ein wasserunlösliches Chitosan-Fasermaterial wird für 2 bis 72 Stunden, vorzugsweise 12 bis 48 Stunden, bei einer Temperatur von -40 °C bis Raumtemperatur in eine wässrige Lösung von NaOH oder KOH eingetaucht und alkalisiert, welche eine Konzentration von 20-50 Gew.-%, vorzugsweise 30-45 Gew.-%, hat, mit dem Gewichtsverhältnis des wasserunlöslichen Chitosan-Fasermaterials zu der wässrigen Lösung von 1: (5-40), vorzugsweise 1: (10-25),
das alkalisierte wasserunlösliche Chitosan-basierte Fasermaterial wird mit halogenierter Essigsäure oder halogeniertem Ethanol oder Propylenoxid oder halogeniertem Propanol oder einer Kombination von halogenierter Essigsäure und halogeniertem Ethanol bei Raumtemperatur in einem Reaktionsmedium für einen Zeitraum von 5 bis 36 Stunden, vorzugsweise 6 bis 24 Stunden umgesetzt, um das entsprechende wasserlösliche Chitosan-Fasermaterial zu ergeben,
wobei das Molverhältnis des wasserunlöslichen Chitosan-basierten Fasermaterials zum Alkali zur halogenierten Essigsäure oder halogeniertem Ethanol oder Propylenoxid oder halogeniertem Propanol oder der Kombination von halogenierter Essigsäure und halogeniertem Ethanol 1: (10-40): (1,1-15), vorzugsweise 1: (20-40): (5-12) beträgt.

4. Verfahren nach Anspruch 3, wobei die halogenierte Essigsäure Chloressigsäure, Bromessigsäure oder Iodessigsäure ist, das halogenierte Ethanol Chlorethanol oder Bromethanol ist, das halogenierte Propanol 1-Chlor-2-propanol oder 1-Brom-2-propanol ist und die Kombination von halogenierter Essigsäure und halogeniertem Ethanol Chloressigsäure und Chlorethanol, Bromessigsäure und Chlorethanol, Chloressigsäure und Bromethanol oder Bromessigsäure und Bromethanol ist.

5. Verfahren nach Anspruch 3, wobei das Reaktionsmedium eines oder mehrere von Methanol, Ethanol, Isopropanol, Butanol, Aceton, Dimethylsulfoxid, Tetrahydrofuran und Dioxan oder eine wässrige Lösung davon ist.

6. Verfahren nach einem der Ansprüche 3-5, wobei die wasserunlösliche Chitosan-basierte Faser in Form von Chitosanfaser, Chitosanfaser-Vliesstoff, Chitosanfaser-Maschenware, geflochtenem Chitosanfaserstoff oder Chitosanfasertuch vorliegt, in dessen Molekülstruktur der Molprozentanteil von deacetyliertem Glycosyl zwischen 0 und 100% des gesamten Glycosyls liegen kann.

7. Verfahren zum Herstellen des wasserlöslichen Chitosan-basierten Fasermaterials nach einem der Ansprüche 1 bis 2, das die folgenden Schritte aufweist:
ein wasserlösliches Chitosan-basiertes Pulver wird in destilliertem Wasser gelöst, um eine wasserlösliche Chitosan-basierte Spinnlösung mit einer Konzentration von 2-10 Gew.-%, vorzugsweise 5-8 Gew.-%, zu ergeben,
die Spinnlösung wird in ein Koagulationsbad bei einer Temperatur von 30-45 °C durch eine Spinn-Düse eingeblasen, um eine wasserlösliche Chitosan-basierte Faser zu bilden, die Faser wird durch eine Capstanwalze in ein Streckbad abgezogen, mittels einer Streckwalze gestreckt und aufgenommen, um ein wasserlösliches Chitosan-basiertes Faserbündel herzustellen,
eine getrocknete wasserlösliche Chitosan-basierte Faser wird durch Entfernen des organischen Lösungsmittels und Trocknen erhalten.

8. Verfahren nach Anspruch 7, wobei das wasserlösliche Chitosan-basierte Pulver ein Pulver von Carboxymethylchitosan, Hydroxypropylchitosan, Hydroxyethylchitosan, Carboxymethylhydroxyethylchitosan oder ein Gemisch davon sein kann und wobei der Substitutionsgrad von Carboxymethyl, Hydroxypropyl oder Hydroxyethyl höher als 60% ist.

9. Verfahren nach Anspruch 7, wobei das Koagulationsbad und das Streckbad ein oder aus einem oder beiden von Ethanol und Aceton oder eine wässrige Lösung davon mit einer Konzentration von mehr als 90 Gew.-% sein können.

10. Verwendung des wasserlöslichen Chitosan-basierten Fasermaterials nach einem der Ansprüche 1-2 bei der Herstellung eines Arzneimittels oder einer medizinischen Vorrichtung zur Hämostase, Wundheilung oder zum Verhindern von Gewebeadhäsion.

## Revendications

1. Matière fibreuse à base de chitosane hydrosoluble, dans laquelle la fibre à base de chitosane hydrosoluble comprend une fibre de chitosane carboxyméthylique, une fibre de chitosane hydroxypropylique, une fibre de chitosane hydroxyéthylique, une fibre de chitosane hydroxyéthylique carboxyméthylique ou un mélange de celles-ci, dans laquelle le chitosane carboxyméthylique a la structure suivante : 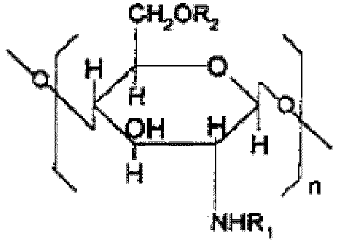 où, R₁ représente H, -COCH₃, -CH₂COONa ou -CH₂COOK ; R₂ représente H, -CH₂COONa ou -CH₂COOK et le degré de substitution de carboxyméthyle est supérieur à 60 % ;
le chitosane hydroxypropylique a la structure suivante : 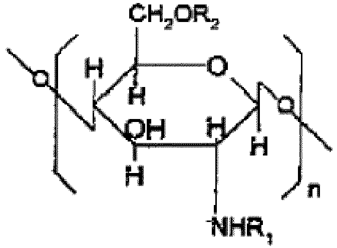 où, R₁ représente H, -COCH₃ ou -CH₂CHOHCH₃ ; R₂ représente H ou -CH₂CHOHCH₃ et le degré de substitution d'hydroxypropyle est supérieur à 60 % ;
le chitosane hydroxyéthylique a la structure suivante : 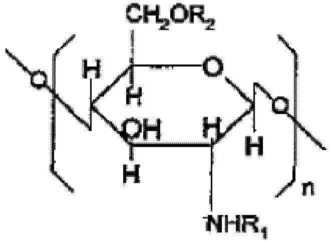 où, R₁ représente H, -COCH₃ ou -CH₂CH₂OH ; R₂ représente H ou -CH₂CH₂OH et le degré de substitution d'hydroxyéthyle est supérieur à 60 % ; ou
le chitosane hydroxyéthylique carboxyméthylique a la structure suivante : 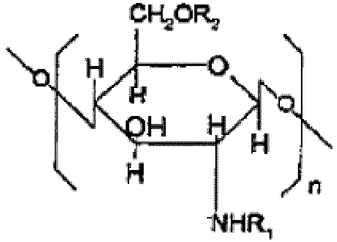 où, R₁ représente H, -COCH₃, -CH₂COONa, -CH₂COOK ou -CH₂CH₂OH ; R₂ représente H, -CH₂COONa, -CH₂COOK ou -CH₂CH₂OH et le degré total de substitution de carboxyméthyle et d'hydroxyéthyle est supérieur à 60 %.

2. Matière fibreuse à base de chitosane hydrosoluble selon la revendication 1, sous la forme de fibre, d'éclat, éponge, ouate, tissu non tissé, tissu tricoté, tissu tressé, tissu tissé.

3. Procédé pour préparer la matière fibreuse à base de chitosane hydrosoluble selon l'une quelconque des revendications 1 et 2, comprenant les étapes suivantes :
une matière fibreuse de chitosane insoluble dans de l'eau est immergée et alcalinisée dans une solution aqueuse de NaOH ou KOH ayant une concentration de 20 à 50 % en poids, de préférence 30 à 45 % en poids, avec le rapport pondéral de la matière fibreuse de chitosane insoluble dans de l'eau sur la solution aqueuse étant de 1:(5-40), de préférence 1:(10-25), à une température allant de -40 °C jusqu'à la température ambiante pendant 2 à 72 heures, de préférence 12 à 48 heures ;
on fait réagir la matière fibreuse à base de chitosane insoluble dans de l'eau alcalinisée avec de l'acide acétique halogéné, ou de l'éthanol halogéné, ou de l'oxyde de propylène, ou du propanol halogéné, ou une combinaison d'acide acétique halogéné et d'éthanol halogéné à température ambiante dans un milieu de réaction pendant une durée de 5 à 36 heures, de préférence 6 à 24 heures, pour donner la matière fibreuse de chitosane hydrosoluble correspondante,
dans lequel le rapport molaire de la matière fibreuse à base de chitosane insoluble dans de l'eau sur la substance alcaline sur l'acide acétique halogéné, ou l'éthanol halogéné, ou l'oxyde de propylène, ou le propanol halogéné, ou une combinaison d'acide acétique halogéné et d'éthanol halogéné est de 1:(10-40):(1.1-15), de préférence 1:(20-40):(5-12).

4. Procédé selon la revendication 3, dans lequel l'acide acétique halogéné est de l'acide chloroacétique, l'acide bromoacétique ou l'acide iodoacétique, l'éthanol halogéné est le chloroéthanol ou le bromoéthanol, le propanol halogéné est du 1-chloro-2-propanol ou du 1-bromo-2-propanol, et la combinaison d'acide acétique halogéné et d'éthanol halogéné est de l'acide chloroacétique et du chloroéthanol, de l'acide bromoacétique et du chloroéthanol, de l'acide chloroacétique et du bromoéthanol, ou de l'acide bromoacétique et du bromoéthanol.

5. Procédé selon la revendication 3, dans lequel le milieu de réaction est un ou plusieurs du méthanol, de l'éthanol, de l'isopropanol, de l'alcool butylique, de l'acétone, du sulfoxyde diméthylique, du tétrahydrofuranne et du dioxane, ou une solution aqueuse de ceux-ci.

6. Procédé selon l'une quelconque des revendications 3 à 5, dans lequel la fibre à base de chitosane insoluble dans de l'eau est sous la forme de fibre de chitosane, de tissu non tissé en fibre de chitosane, de tissu tricoté en fibre de chitosane, de tissu tressé en fibre de chitosane ou de tissu tissé en fibre de chitosane, dans la structure moléculaire de laquelle le pourcentage molaire de glycosyle désacétylé peut être entre 0 et 100 % du glycosyle total.

7. Procédé de préparation de la matière fibreuse à base de chitosane hydrosoluble selon l'une quelconque des revendications 1 - 2, comprenant les étapes suivantes :
une poudre à base de chitosane hydrosoluble est dissoute dans de l'eau distillée pour donner une solution de filage à base de chitosane hydrosoluble ayant une concentration de 2 à 10 % en poids, de préférence 5 à 8 % en poids ;
la solution de filage est soufflée dans un bain de coagulation à une température de 30 à 45 °C via une filière de filage, pour former une fibre à base de chitosane hydrosoluble ;
la fibre est tirée par un rouleau tenseur dans un bain d'étirement, étirée par un rouleau d'étirage et extraite, pour produire un paquet de fibres à base de chitosane hydrosoluble ;
une fibre à base de chitosane hydrosoluble séchée est obtenue par enlèvement de solvant organique et séchage.

8. Procédé selon la revendication 7, dans lequel la poudre à base de chitosane hydrosoluble peut être une poudre de chitosane carboxyméthylique, de chitosane hydroxypropylique, de chitosane hydroxyéthylique, de chitosane hydroxyéthylique carboxyméthylique ou un mélange de ceux-ci, et le degré de substitution de carboxyméthyle, d'hydroxypropyle ou d'hydroxyéthyle est supérieur à 60%.

9. Procédé selon la revendication 7, dans lequel le bain de coagulation et le bain d'étirement peuvent être l'un ou les deux d'éthanol et d'acétone, ou une solution aqueuse de ceux-ci ayant une concentration supérieure à 90 % en poids.

10. Utilisation de la matière fibreuse à base de chitosane hydrosoluble selon l'une quelconque des revendications 1 - 2 dans la production d'un médicament ou d'un dispositif médical pour hémostase, cicatrisation ou prévention d'adhérence de tissu.
